# EUROPEAN PATENT SPECIFICATION

(11) **EP 1 707 220 B1**
(45) Date of publication and mention of the grant of the patent: **20.02.2008**
(21) Application number: 06006315.3
(22) Date of filing: 27.03.2006
(51) Int. Cl.: A61L 2/22

(54) **Weak acid solution nebulizer with cleaning function**
Vernebler für eine schwach saure Lösung mit Reinigungsfunktion
Nébulisateur pour une solution faiblement acide avec une fonction de nettoyage

(30) Priority: 28.03.2005 JP 2005091001
(43) Date of publication of application: 04.10.2006
(73) Proprietor: Toray Ireeve Corporation, Shinjuku-ku Tokyo (JP); Hokuetsu Co., Ltd., Yamato-shi Kanagawa (JP); HIROSE ELECTRIC CO., LTD., Shinagawa-ku Tokyo 141 (JP)
(72) Inventor: Nomura, Akio c/o Toray Ireeve Corporation, Skinjuku, Tokyo (JP); Katsura, Tadao c/o Toray Ireeve Corporation, Skinjuku, Tokyo (JP); Mita, Kosuke c/o Toray Ireeve Corporation, Skinjuku, Tokyo (JP); Suzuki, Masaki c/o Hokuetsu Co., Ltd., Yamato-shi, Kanagawa (JP); Sato, Hirotsugu c/o HIROSE ELECTRIC CO., LTD., Shinagawa-ku, Tokyo (JP); Nakagawa, Tetsuo c/o HIROSE ELECTRIC CO., LTD., Shinagawa-ku, Tokyo (JP); Nemoto, Takashi c/o HIROSE ELECTRIC CO., LTD., Shinagawa-ku, Tokyo (JP); Takano, Kazuhiko c/o HIROSE ELECTRIC CO., LTD., Shinagawa-ku, Tokyo (JP)
(74) Representative: Müller-Boré & Partner Patentanwälte

(56) References cited:
- US-A1- 2004 091 389
- PATENT ABSTRACTS OF JAPAN vol. 2003, no. 10, 8 October 2003 (2003-10-08) & JP 2003 169842 A (SONY CORP; VTA KK), 17 June 2003 (2003-06-17) & JP 2003 169842 A (SONY CORP; VTA KK) 17 June 2003 (2003-06-17)

## Description

This invention relates to a slightly acid solution nebulizer with a cleaning function which can nebulize, for example, a hypochlorous acid solution by the effect of ultrasound.

A slightly acid solution such as a hypochlorous acid solution is known to have strong sterilizing and deodorizing capabilities. In the food industry and healthcare industry, hospital rooms, tableware and the like are sterilized and deodorized by use of the characteristics of the slightly acid solution. As use of the slightly acid solution has been spread, various improvements have been made on apparatuses which produce the slightly acid solution. For example, in Japanese Patent Laid-Open Publication Nos. 169842/2003 and 197689/2000, apparatuses improved to discharge the slightly acid solution over a wide range are disclosed.

JP 169842/2003 describes a sterilizing method, wherein ultrasonic waves are acted by an ultrasonic vibrator transducer on an aqueous hypochlorous acid solution within an aqueous hypochlorous acid solution storage section and the particulates of the aqueous hypochlorous acid solution are generated in a particulate generating section and are released into the atmosphere from an atomizing port by an induction pipeline, and a sterilizing apparatus which is suited for applying the above method.

Although lower than the acidity of acid solution, the acidity of the slightly acid solution is higher than that of normal water. Consequently, the slightly acid solution is highly liable to corrode the apparatuses. In particular, corrosion of reservoir which reserves relatively high concentration acidic liquid which is a source for producing the slightly acid solution, e.g. diluted hydrochloric acid, is a problem. In general, when the apparatuses are used in industries, it is considered as a daily task to clean the apparatuses with a large quantity of water obtained directly from a faucet or the like, and the task is not so difficult. However, when the apparatuses are used in households, it is often difficult to use water obtained from a faucet directly on the apparatuses, and maintenance of the apparatuses is liable to become cumbersome as compared with when the apparatuses are used in industries.

### Patent Literature 1

Japanese Patent Laid-Open Publication No. 169842/2003

### Patent Literature 2

Japanese Patent Laid-Open Publication No. 197689/2000

The present invention has been conceived to solve the above problems of the prior art. An object of the present invention is to provide a slightly acid solution nebulizer capable of cleaning a reservoir that reserves, for example, diluted hydrochloric acid.

The present invention is a slightly acid solution nebulizer with a cleaning function which comprises:
a gas generator,
a reservoir device,
a nebulization device, and
a cleaning device,
wherein
the gas generator generates chlorine gas by electrolyzing diluted hydrochloric acid,
the reservoir device reserves water to be mixed with the chlorine gas to produce a hypochlorous acid solution,
the nebulization device nebulizes the hypochlorous acid solution, and
the cleaning device cleans the gas generator by leading the water reserved in the reservoir device to the gas generator after production of the hypochlorous acid solution.

In the above nebulizer, the above gas generator may be degassed after cleaned.

In the above nebulizer, the above reservoir device can be detached from the above nebulizer.

In the above nebulizer, the above nebulization device may have an ultrasonic vibrator, and the ultrasonic vibrator may have a partition therearound to separate itself from the slightly acid solution.

In the above nebulizer, the above nebulization device may have an ultrasonic vibrator, and the ultrasonic vibrator may have a vinyl thin film on its portion that contacts the slightly acid solution.

In the above nebulizer, a lubricant may be applied between the above thin film and the above ultrasonic vibrator.

In the above nebulizer, an inlet for charging the diluted hydrochloric acid into the gas generator may have a valve for preventing gas collected inside the gas generator from leaking to the outside, and the above valve may have a cross slit.

In the above nebulizer, the above nebulization device may have an ultrasonic vibrator, and an upper limit sensor and a lower limit sensor may be provided that retain the amount of the hypochlorous acid solution within a predetermined range when the solution is vibrated by the ultrasonic vibrator.

Since the electrolytic device can be cleaned after production of the hypochlorous acid solution, maintenance of the apparatus can be facilitated.
Fig. 1 is an external perspective view of a main unit and a main unit cover.
Fig. 2 is an external perspective view of the main unit after members detachable from the main unit are detached.
Fig. 3 is a schematic front view of the internal structure of the present apparatus.
Fig. 4 is a schematic side view of the internal structure of the present apparatus.

Hereinafter, the present invention will be described with reference to the drawings.

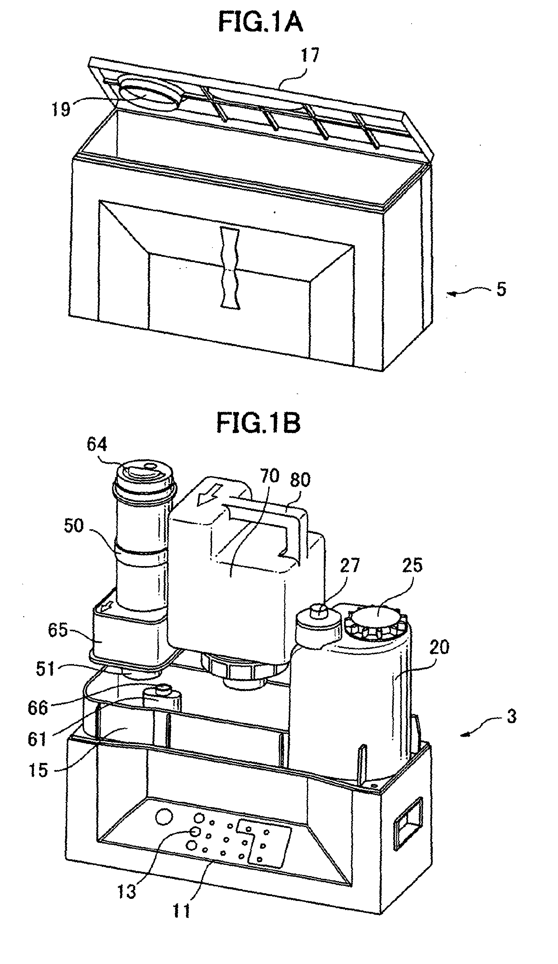

Figs. 1 to 4 show an ultrasonic nebulizer according to one preferred embodiment of the present invention. This apparatus 1 may be formed in a relatively small size so that it can be placed in a room of general household.

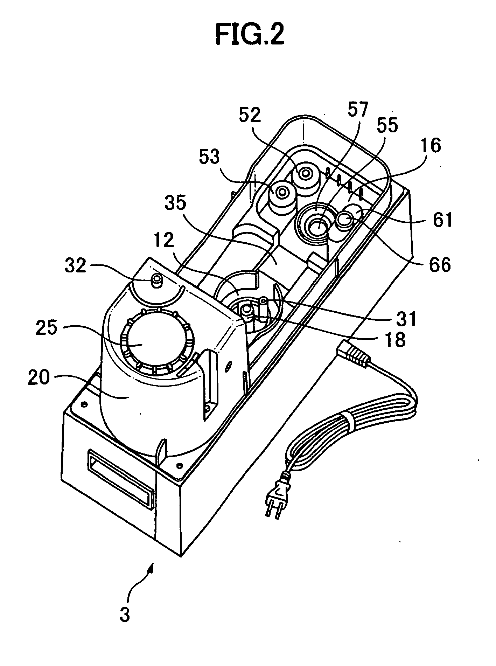

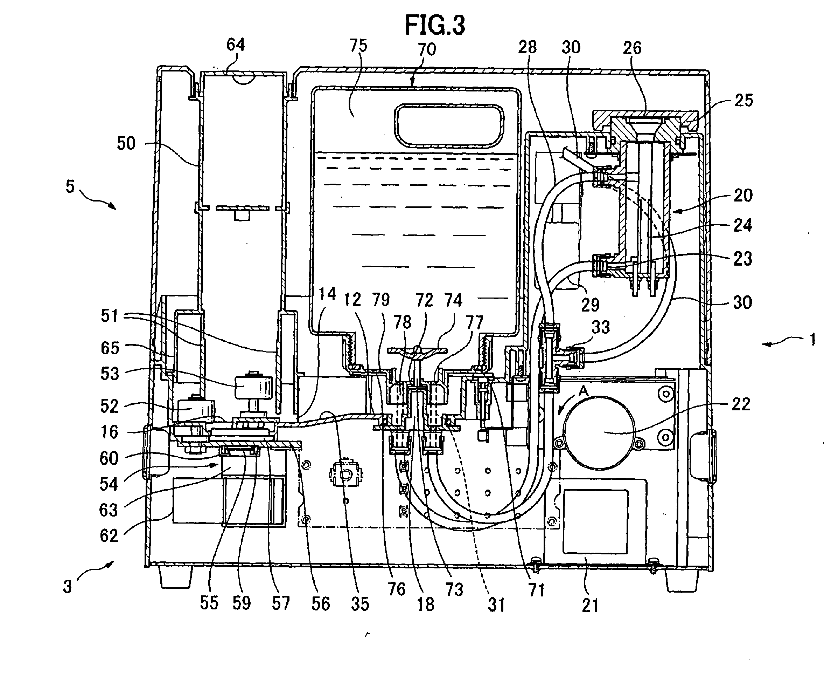

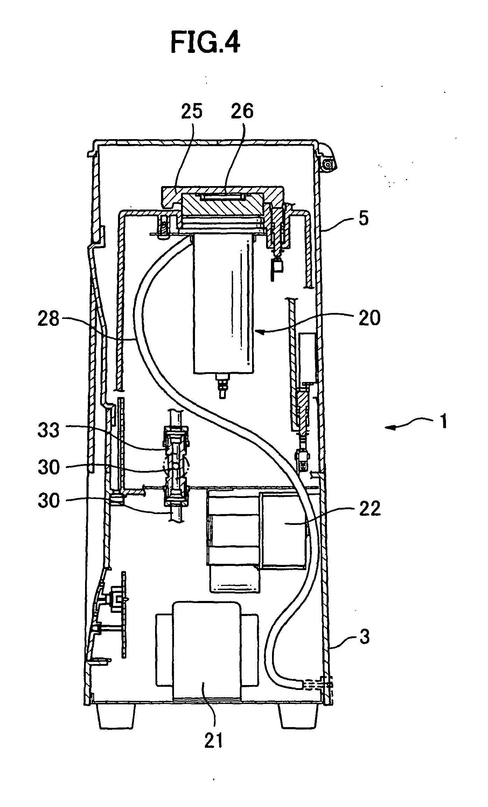

The apparatus 1 can be separated into a main unit 3 and a main unit cover 5 that can be attached detachably such that it covers a portion of the upper portion of the main unit 3. Fig. 1A shows an external perspective view of the main unit cover 5, and Fig. 1B shows an external perspective view of the main unit 3. Fig. 2 shows an external perspective view of the main unit 3 with members detachable from the main unit 3 detached. Fig. 3 is a schematic front view of the internal structure of the apparatus 1, and Fig. 4 is a schematic side view thereof.

An operation panel 11 is provided on an outer surface of the main unit 3. A user can perform production of a hypochlorous acid solution, nebulization of a produced hypochlorous acid solution, cleaning of the nebulizer or the like, by pressing a predetermined push-button switch 13 provided on the operation panel 11, for example. Further, the user can select the amount of a hypochlorous acid solution to be nebulized stepwise or set nebulization time by the hour, for example. In particular, cleaning of the nebulizer may be performed automatically without operation of the push-button switch by the user.

A top cover 17 is provided on the top of the main unit cover 5. By providing the top cover 17, the user can access the internal members of the main unit, e.g. an electrolytic device 20, a nebulization cylinder 50 and a reservoir tank 70, easily, without detaching the main unit cover 5 from the main unit 3. An opening 19 is formed in a portion of the top cover 17 to allow a nebulization nozzle 64 provided on the top of the nebulization cylinder 50 to be exposed therefrom.

While the nebulization cylinder 50 and the reservoir tank 70 are provided such that they can be detached from the main unit 3, the electrolytic device 20 is completely fixed to the main unit 3. The user can attach the nebulization cylinder 50 and the reservoir tank 70 to the main unit 3 or detach the cylinder 50 and the tank 70 from the main unit 3 easily by grabbing a handle 80 and bringing up or down the cylinder 50 and the tank 70 along the installation frame 15 of the main unit 3.

To produce a hypochlorous acid solution, for example, 0.1 to 10%, 5 to 150 cc, specifically, 1.1%, 7 cc of diluted hydrochloric acid is charged into the electrolytic device 20 in advance. At that time, to prevent gas collected in the electrolytic device 20 by previous use from leaking to the outside, a rubber valve (not shown) may be provided right underneath the electrolytic cap 25 of an inlet 26. Further, a cross slit may be made in the rubber valve so that diluted hydrochloric acid is charged into the electrolytic device 20 easily and is hardly spilled out. The slit is also useful for preventing gas remaining in the electrolytic device 20 from leaking to the outside and making the user uncomfortable when he opens the electrolytic cap 25.

In the electrolytic device 20, a pair of electrodes 24 are disposed, facing each other. The electrodes 24 may be formed by coating titanium with platinum, for example. Chlorine gas can be produced by electrolyzing diluted hydrochloric acid charged into the electrolytic device 20 by the electrodes 24. The chlorine gas is then mixed with water (tap water) reserved in advance in the reservoir tank 70. As a result, for example, a specific hypochlorous acid solution having a pH of 4.0 to 7.5 (preferably a pH of 5.0 to 6.5) and a chlorine concentration of 1 to 60 ppm (preferably 10 to 30 ppm) is produced in the reservoir tank 70. For example, a 2-L hypochlorous acid solution can be produced from 1.1%, 7 cc of diluted hydrochloric acid.

The reservoir tank 70 can be separated into a tank body 75 and a tank cover 76. Tap water is filled in the reservoir tank 70 with the tank cover 76 removed. The reservoir tank 70 can be attached to and detached from the main unit 3 freely with the tank cover 76 secured to the tank body 75. Upon attachment to or detachment from the main unit 3, more specifically, when the reservoir tank 70 is detached from the main unit 3, the tank body 75 is completely sealed by the tank cover 76 via a packing 71. Meanwhile, when the reservoir tank 70 is attached to the main unit 3, a tank plug 74 is moved to the tank body 75 side (Fig. 3 shows the state of the plug 74 after moved), against the elastic force of a tank plug spring 72 provided along a tank plug shaft 73, by the action of a vertical gating shaft 18 provided on the main unit, whereby the opening 77 of the tank cover 76 is opened. As a result, water in the reservoir tank 70 can be mixed with the outside air (gas), and the water in the reservoir tank 70 can be discharged to the outside. When the reservoir tank 70 is attached to the main unit 3, a chlorine inlet 31 extending parallel to the vertical gating shaft 18 from the main unit side reaches the vicinity of the opening 77. Chlorine gas produced in the electrolytic device 20 is led directly to the water in the reservoir tank 70, via the chlorine inlet 31 and a gas feeding and cleaning solution backflowing tube 28 which connects between the chlorine inlet 31 and the electrolytic device 20. As a result, the chlorine gas is mixed with the water in the reservoir tank 70, whereby the water in the reservoir tank 70 is turned into a hypochlorous acid solution. Although the thus produced hypochlorous acid solution may be nebulized by use of the nebulization cylinder 50, it is also possible that the reservoir tank 70 is detached from the main unit 3 and the hypochlorous acid solution is used as it is. To make it easy to use the hypochlorous acid solution as it is, for example, a funnel 27 mounted in a funnel mounting portion 32 (refer to Fig. 2) provided on the top of the electrolytic device 20 may be attached to the plug portion 79 of the tank cover 76 to transfer the hypochlorous acid solution in the reservoir tank 70 into another container such as a PET bottle easily.

Between the electrolytic device 20 and the reservoir tank 70, an electrolytic device liquid drainage tube 29 and an electrolytic device gas exhaust tube 30 are provided, in addition to the gas feeding and cleaning solution backflowing tube 28. These tubes 28, 29 and 30 are used to clean the electrolytic device 20 after production of the hypochlorous acid solution. For example, the electrodes 24 in the electrolytic device 20 which are formed by coating titanium with platinum are highly liable to corrode. For this reason, it is preferable to clean the electrolytic device 20 after production of the hypochlorous acid solution

As shown in the drawing, the electrolytic device liquid drainage tube 29 is connected to a liquid outlet 78 extending from the main unit 3 to the vicinity of the opening 77 of the reservoir tank 70. Meanwhile, the electrolytic device gas exhaust tube 30 is connected to the gas feeding and cleaning solution backflowing tube 28 via a branching section 33 provided in the middle of the tube 28. Although it is not necessarily clear from the drawing, the front end of the electrolytic device gas exhaust tube 30 is opened to outside air in such a manner that it can be controlled by an electromagnetic valve (not shown).

After production of the hypochlorous acid solution, the rotary (not shown) of pump unit 22 rotates in the direction indicated by the arrow A in the drawing, automatically or in response to an operation by the user. This rotation pushes the electrolytic device liquid drainage tube 29 in the direction indicated by the arrow A in the same drawing. As a result, diluted hydrochloric acid in the electrolytic device 20 together with other liquids is discharged from the bottom outlet 23 of the electrolytic device 20 to the reservoir tank 70 through the electrolytic device liquid drainage tube 29 and the liquid outlet 78. Meanwhile, in the gas feeding and cleaning solution backflowing tube 28, the hypochlorous acid solution in the reservoir tank 70 flows backward, in response to the rotation of the rotary (i.e. in response to discharge of the liquid from the electrolytic device 20 to the reservoir tank 70). The backflowed hypochlorous acid solution drips to the electrolytic device 20 from above the electrodes 24 via the gas feeding and cleaning solution backflowing tube 28. Thus, by rotating the rotary of the pump unit 22, high concentration diluted hydrochloric acid in the electrolytic device 20 can be discharged from the electrolytic device 20 via the electrolytic device liquid drainage tube 29, and low concentration diluted hydrochloric acid (hypochlorous acid solution) in the reservoir tank 70 can be taken into the electrolytic device 20 via the gas feeding and cleaning solution backflowing tube 28. As a result, acidity of the inside of the electrolytic device 20 is reduced; that is, the inside of the electrolytic device 20 is cleaned. After completion of these cleaning operations, the electrolytic device gas exhaust tube 30 is opened by the action of the electromagnetic valve, and the pump unit 22 is operated to discharge diluted hydrochloric acid (hypochlorous acid solution) collected inside the electrolytic device 20 and the gas feeding and cleaning solution backflowing tube 28 to the outside. Thereby, when electrolysis is conducted next time, diluted hydrochloric acid does not spill out of the electrolytic device 20 and the concentration does not change with respect to a specified value when diluted hydrochloric acid is added.

A nebulization unit for nebulizing a hypochlorous acid solution in the air primarily comprises the nebulization cylinder 50, an ultrasonic vibrator 55, and an air blowing section 61.

The nebulization cylinder 50 can be attached to and detached from the main unit 3 freely, and its base is formed as a rectangular frame 65 in accordance with the installation frame 15 of the main unit 3. When the nebulization cylinder 50 is mounted on the main unit 3, a little gap 14 is formed between the rectangular frame 65 and a main unit bottom 12 on the tank side. A hypochlorous acid solution having flowed out of the reservoir tank 70 via the opening 77 of the tank cover 76 flows on a main unit inclined plane 35 and then flows and pools in a main unit bottom 16 on the nebulization cylinder side which is lower than the main unit bottom 12 on the tank side through the gap 14.

The level of the hypochlorous acid solution pooled in the vicinity of the main unit bottom 16 can be detected by a lower limit float switch 52 and an upper limit float switch 53. When the level of the hypochlorous acid solution exceeds limits set by the lower limit float switch 52 and the upper limit float switch 53, in other words, when the amount of the hypochlorous acid solution is too small or too large, a predetermined signal is sent to a user, and the level of the solution is also adjusted automatically. Further, the lower limit float switch 52 that serves as a lower limit sensor is useful for preventing the ultrasonic vibrator 55 from being broken by its own vibration when the nebulizer is operated without the hypochlorous acid solution, and the upper limit float switch 53 that serves as an upper limit sensor is useful for preventing the hypochlorous acid solution from flowing out over the installation frame 15 of the main unit 3. By keeping the upper and lower limits of the level of the solution and keeping the solution level within a predetermined range by using these lower limit float switch 52 and upper limit float switch 53, the slightly acid solution can be retained in such an optimum amount that the nebulization cylinder 50 operates efficiently.

Upon nebulization, the hypochlorous acid solution pooled in the main unit bottom 16 is subjected to ultrasound generated by the ultrasonic vibrator 55. By the effect of the ultrasound, fine particles of the hypochlorous acid solution are produced from the surface of the solution. Then, these fine particles are turned into mist by blast airstream generated in the air blowing section 61. The ultrasonic vibrator 55 is housed in a case 60 which is provided under the main unit bottom 16 such that its perimeter is supported by a packing 59. In addition, the perimeter of the case 60 is covered by an O ring 57. To release heat from the ultrasonic vibrator 55, a cooling plate 56 may be provided at a position close to the ultrasonic vibrator 55. The ultrasonic vibrator 55 is highly liable to corrode since it is constantly exposed to water. The problem is more severe in the present invention since the present apparatus uses an acid solution. Conventional ultrasonic vibrators use a metal sheet (such as an SUS sheet) to prevent corrosion and are plated with gold or coated with fluorine to enhance corrosion resistance. However, these conventional methods do not exert sufficient corrosion resistance to a hypochlorous acid solution used in the present apparatus. Further, in addition to the problem of corrosion, the ultrasonic vibrator 55 also has a problem that when sound energy generated by the vibrator 55 is not transferred directly to a medium having mass such as water, all sound energy is concentrated on the vibrator 55 and the vibrator 55 is broken by its own action. To solve these problems, in the present invention, in at least a portion of the ultrasonic vibrator 55 which contacts the hypochlorous acid solution, a lubricant such as grease including fluorine grease and silicone grease is applied on the surface of a metal sheet (such as an SUS sheet), and a vinyl thin film such as vinylidene chloride, polyethylene or polypropylene is then applied. Thereby, corrosion resistance can be improved significantly while the reliability of the operation is maintained.

Another problem associated with the ultrasonic vibrator 55 is that the hypochlorous acid solution is liable to be degraded by vibration. When the hypochlorous acid solution is vibrated, chlorine gas dissolved in the hypochlorous acid solution is released, whereby the effective chlorine concentration is lowered. To prevent this problem, it is desirable to minimize vibration of the ultrasonic vibrator 55 that is transmitted to the hypochlorous acid solution. For this reason, in the present apparatus, a circular wall 51 that matches the shape of the ultrasonic vibrator 55 is formed to surround the portion above the ultrasonic vibrator 55. Thus, a partition is formed between the hypochlorous acid solution and the ultrasonic vibrator, and the influence of vibration of the ultrasonic vibrator 55 on the hypochlorous acid solution can be reduced.

The frequency of vibration of the ultrasonic vibrator 55 is not particularly limited but is preferably relatively high frequency. For example, while the frequency of an ultrasonic vibrator used in a conventional humidifier is around 1.6 MHz, the ultrasonic vibrator 55 of the present apparatus uses a frequency of around 2.2 to 2.6 MHz (more specifically, 2.4 MHz) . By use of relatively high frequency, water to be nebulized can be made fine. For example, while a general humidifier can produce water droplets of merely around 10 microns, fine water droplets of around 1 to 5 microns can be produced by use of such relatively high frequency as described above. Water droplets of this size are sufficient to reach and eliminate germs. Further, by making water droplets fine as described above, time in which the water droplets remain in the air can be increased, and the sterilizing capability is not deteriorated. Consequently, sterilization can be performed efficiently with a small amount of a hypochlorous acid solution.

The fine particles of the hypochlorous acid solution which have been generated by the ultrasonic vibrator 55 are then spurted to the outside from the nebulization nozzle 64 provided on the top of the nebulization cylinder 50 as a misty hypochlorous acid solution by the action of the air blowing section 61. The air blowing section 61 primarily comprises a DC fan 62 and a duct 63 which are disposed in the base of the main unit. Wind generated by the DC fan 62 is led to an air outlet 66 through the duct 63 and spurted from the small air outlet 66. This airstream with the fine particles is transferred in a mist form, whereby the hypochlorous acid solution is nebulized.

Finally, a description will be given to an example of use of the present apparatus. As the preparatory stage, diluted hydrochloric acid is charged into the electrolytic device 20, and the reservoir tank 70 is filled with tap water and set in the main unit 3. After preparation, an electric current is passed through the electrodes 24 to electrolyze the diluted hydrochloric acid in the electrolytic device 20, thereby generating chlorine gas from the electrolytic device 20. The generated chlorine gas is led to the reservoir tank 70 through the gas feeding and cleaning solution backflowing tube 28, the chlorine inlet 31 and the opening 77 and mixed with the water in the reservoir tank 70. As a result, a hypochlorous acid solution is produced in the reservoir tank 70. The hypochlorous acid solution can be used as it is by detaching the reservoir tank 70 from the main unit 3 or can be nebulized into the air by the ultrasonic vibrator 55 and the air blowing section 61 after moved to the main unit bottom 16.

After use, the electrolytic device 20 is cleaned. This cleaning may be started automatically or in response to operation of the button by a user. First, the diluted hydrochloric acid solution (hypochlorous acid solution) remaining in the electrolytic device 20 is discharged to the outside. This is done by sending the remaining diluted hydrochloric acid solution (hypochlorous acid solution) to the reservoir tank 70 through the electrolytic device liquid drainage tube 29 connected to the bottom of the electrolytic device 20 by rotating the rotary (not shown) of the pump unit 22 in the direction indicated by the arrow A in the drawing. Along with this operation, the air pressure inside the electrolytic device 20 decreases. By the decrease in the air pressure, the liquid in the reservoir tank 70 flows back to the electrolytic device 20 through the gas feeding and cleaning solution backflowing tube 28. As a result, the backflowed hypochlorous acid solution drips to the inside of the electrolytic device 20 from above the electrodes 24. Since the liquid in the reservoir tank 70 has been diluted with a large amount of water, the acidity thereof is significantly lower than that of the diluted hydrochloric acid which has remained in the electrolytic device 20. Therefore, by continuing this operation over a predetermined time, the acidity of the electrolytic device 20 can be decreased. Further, as an additional stage, gas remaining in the electrolytic device 20 may also be discharged. The rotary of the pump unit 22 is rotated even after cleaning, and an electromagnetic valve (not shown) is operated to open the branching section 33. As a result, the hypochlorous acid solution having backflowed from the reservoir tank 70 returns to the reservoir tank 70, whereby the electrolytic device 20 and the gas feeding and cleaning solution backflowing tube 28 can be emptied. This can prevent diluted hydrochloric acid from spilling out of the electrolytic device or prevent the concentration from changing with respect to a specified value by adding diluted hydrochloric acid when electrolysis is conducted next time.

## Claims

1. A slightly acid solution nebulizer (1) with a cleaning function, comprising:
a gas generator (20),
a reservoir device (70),
a nebulization device (50), and
a cleaning device,
wherein
the gas generator (20) is adapted to generate chlorine gas by electrolyzing diluted hydrochloric acid,
the reservoir device (70) is adapted to reserve water to be mixed with the chlorine gas to produce a hypochlorous acid solution,
the nebulization device (50) is adapted to nebulize the hypochlorous acid solution, and
the cleaning device is adapted to clean the gas generator by leading the water reserved in the reservoir device (70) to the gas generator (20) after production of the hypochlorous acid solution.

2. The nebulizer of claim 1, wherein the gas generator is adapted to be degassed after cleaned.

3. The nebulizer of claim 1 or 2, wherein the reservoir device can be detached from the nebulizer.

4. The nebulizer of any of claims 1 to 3, wherein the nebulization device has an ultrasonic vibrator, and the ultrasonic vibrator has a partition therearound to separate itself from the hypochlorous acid solution.

5. The nebulizer of any of claims 1 to 4, wherein the nebulization device has an ultrasonic vibrator, and the ultrasonic vibrator has a vinyl thin film on its portion that contacts the hypochlorous acid solution.

6. The nebulizer of claim 5, wherein a lubricant is applied between the thin film and the ultrasonic vibrator.

7. The nebulizer of any of claims 1 to 6, wherein an inlet for charging the diluted hydrochloric acid into the gas generator has a valve for preventing gas collected inside the gas generator from leaking to the outside, and the valve has a cross slit.

8. The nebulizer of any of claims 1 to 7, wherein the nebulization device has an ultrasonic vibrator, and an upper limit sensor and a lower limit sensor are provided in order to retain the amount of the hypochlorous acid solution within a predetermined range when the solution is vibrated by the ultrasonic vibrator.

## Patentansprüche

1. Zerstäuber (1) für eine schwach saure Lösung mit einer Reinigungsfunktion, umfassend:
einen Gasgenerator (20),
eine Reservoirvorrichtung (70),
eine Zerstäubungsvorrichtung (50) und
eine Reinigungsvorrichtung,
wobei der Gasgenerator (20) angepaßt ist, um Chlorgas durch Elektrolysieren einer verdünnten Chlorwasserstoffsäure zu erzeugen,
die Reservoirvorrichtung (70) angepaßt ist, um Wasser aufzubewahren, welches mit dem Chlorgas zu vermischen ist, um eine Hypochlorsäurelösung herzustellen,
die Zerstäubungsvorrichtung (50) angepaßt ist, um die Hypochlorsäurelösung zu zerstäuben, und
die Reinigungsvorrichtung angepaßt ist, um den Gasgenerator durch Führen des in der Reservoirvorrichtung (70) aufbewahrten Wassers zu dem Gasgenerator (20) nach Herstellung der Hypochlorsäurelösung zu reinigen.

2. Zerstäuber nach Anspruch 1, wobei der Gasgenerator angepaßt ist, um nach Reinigung entgast zu werden.

3. Zerstäuber nach Anspruch 1 oder 2, wobei die Reservoirvorrichtung von dem Zerstäuber abgenommen werden kann.

4. Zerstäuber nach einem der Ansprüche 1 bis 3, wobei die Zerstäubungsvorrichtung einen Ultraschallvibrator aufweist und der Ultraschallvibrator eine Abtrennung rundherum aufweist, um ihn von der Hypochlorsäurelösung zu trennen.

5. Zerstäuber nach einem der Ansprüche 1 bis 4, wobei die Zerstäubungsvorrichtung einen Ultraschallvibrator aufweist und der Ultraschallvibrator einen dünnen Vinylfilm an dessen Bereich aufweist, der die Hypochlorsäurelösung kontaktiert.

6. Zerstäuber nach Anspruch 5, wobei ein Schmiermittel zwischen dem dünnen Film und dem Ultraschallvibrator angebracht ist.

7. Zerstäuber nach einem der Ansprüche 1 bis 6, wobei ein Einlaß zum Beschikken der verdünnten Chlorwasserstoffsäure in den Gasgenerator ein Ventil aufweist, um zu verhindern, daß Gas, welches sich in dem Gasgenerator angesammelt hat, zu der Außenseite ausfließt, und das Ventil einen Kreuzschlitz aufweist.

8. Zerstäuber nach einem der Ansprüche 1 bis 7, wobei die Zerstäubungsvorrichtung einen Ultraschallvibrator aufweist und ein Sensor für die obere Grenze und ein Sensor für die untere Grenze bereitgestellt sind, um die Menge an Hypochlorsäurelösung in einem vorbestimmten Bereich zu halten, wenn die Lösung durch den Ultraschallvibrator vibriert wird.

## Revendications

1. Nébuliseur de solution légèrement acide
(1) ayant une fonction de nettoyage, comportant :
un générateur de gaz (20),
un dispositif formant réservoir (70),
un dispositif de nébulisation (50), et
un dispositif de nettoyage,
dans lequel
le générateur de gaz (20) est adapté pour générer du chlore gazeux en électrolysant de l'acide chlorhydrique dilué,
le dispositif formant réservoir (70) est adapté pour conserver de l'eau devant être mélangée avec le chlore gazeux afin de produire une solution d'acide hypochloreux,
le dispositif de nébulisation (50) est adapté pour nébuliser la solution d'acide hypochloreux, et
le dispositif de nettoyage est adapté pour nettoyer le générateur de gaz en conduisant l'eau entreposée dans le dispositif formant réservoir (70) vers le générateur de gaz (20) après la production de la solution d'acide hypochloreux.

2. Nébuliseur selon la revendication 1, dans lequel le générateur de gaz est adapté pour être dégazé après avoir été nettoyé.

3. Nébuliseur selon la revendication 1 ou 2, dans lequel le dispositif formant réservoir peut être détaché du nébuliseur.

4. Nébuliseur selon l'une quelconque des revendications 1 à 3, dans lequel le dispositif de nébulisation a un vibreur ultrasonore, et le vibreur ultrasonore a une séparation autour de celui-ci pour se séparer lui-même de la solution d'acide hypochloreux.

5. Nébuliseur selon l'une quelconque des revendications 1 à 4, dans lequel le dispositif de nébulisation a un vibreur ultrasonore, et le vibreur ultrasonore a un film mince de vinyle qui vient en contact avec la solution d'acide hypochloreux.

6. Nébuliseur selon la revendication 5, dans lequel un lubrifiant est appliqué entre le film mince et le vibreur ultrasonore.

7. Nébuliseur selon l'une quelconque des revendications 1 à 6, dans lequel une entrée pour charger l'acide chlorhydrique dilué dans le générateur de gaz a une vanne pour empêcher le gaz recueilli à l'intérieur du générateur de gaz de s'échapper vers l'extérieur, et la vanne a une fente transversale.

8. Nébuliseur selon l'une quelconque des revendications 1 à 7, dans lequel le dispositif de nébulisation a un vibreur ultrasonore, et un capteur de limite supérieure et un capteur de limite inférieure sont fournis pour retenir la quantité de la solution d'acide hypochloreux dans une plage prédéterminée lorsque la solution est mise à vibrer par un vibreur ultrasonore.
